Europäisches Patentamt

European Patent Office

Office européen des brevets

(11) Veröffentlichungsnummer: **0 022 426**
B1

(12) **EUROPÄISCHE PATENTSCHRIFT**

(45) Veröffentlichungstag der Patentschrift: **10.09.86**

(21) Anmeldenummer: **80810214.9**

(22) Anmeldetag: **30.06.80**

(51) Int. Cl.⁴: **A 61 K 9/06**, A 61 K 31/715, A 61 K 35/84

(54) **Nasalpräparate und Verfahren zu deren Herstellung.**

(30) Priorität: **04.07.79 CH 6240/79**

(43) Veröffentlichungstag der Anmeldung:
**14.01.81 Patentblatt 81/02**

(45) Bekanntmachung des Hinweises auf die
Patenterteilung:
**10.09.86 Patentblatt 86/37**

(84) Benannte Vertragsstaaten:
**BE CH DE FR GB IT LI LU NL SE**

(56) Entgegenhaltungen:
**DE-A-2 655 844**
**FR-A-2 081 341**
**FR-A-2 293 214**
**FR-A-2 312 261**
**FR-A-2 385 734**

(73) Patentinhaber: **KUREHA CHEMICAL INDUSTRY CO., LTD.**
**9-11, Horidome-cho, 1-chome Nihonbashi Chuo-ku Tokyo 103 (JP)**

(72) Erfinder: **Schmidt-Ruppin, Karl Heinz, Dr.**
**Im Baumgarten 5**
**CH-4144 Arlesheim (CH)**

(74) Vertreter: **Myerscough, Philip Boyd et al**
**J.A.Kemp & Co. 14, South Square Gray's Inn**
**London, WC1R 5EU (GB)**

Courier Press, Leamington Spa, England.

## Beschreibung

Die vorliegende Erfindung betrifft neue Naselpräparate zur Prophylaxe gegen Infektionen durch Viren des Respirationstraktes, vor allem gegen Influenza Viren A und B, Verfahren zur deren Herstellung sowie die Verwendung dieser Präparate.

Es wurde überraschenderweise gefunden, dass das weiter unten näher beschriebene stickstoffhaltige Polysaccharid Krestin bei intranasaler Applikation eine mehrere Tage lang dauernde prophylaktische Wirkung gegenüber Infektionen durch Viren des Respirationstraktes, insbesondere Influenza A- und B-Viren ausübt und bei Mischung mit Viren des Respirationstraktes die Infektiosität der betreffenden Viren aufhebt, ohne deren Fähigkeit zur Anregung der Antikörperbildung zu beeinträchtigen.

Ein Gegenstand vorliegender Erfindung sind deshalb Nasalpräparate zur Prophylaxe gegen Infektionen durch Viren des Respirationstraktes, die als Wirkstoff 0.2—4% Krestin enthalten. Die erfindungsgemässen Präparate sind insbesondere übliche Arzneiformen für die intranasale Anwendung.

Die erfindungsgemässen Nasalpräparate werden dadurch hergestellt, dass man Krestin in einer eine Konzentration von 0.2—4% ergebenden Menge in Wasser löst, gegebenenfalls übliche Zusatzstoffe beifügt und gewünschtenfalls die erhaltene Lösung in zur nasalen Applikation einer Flüssigkeit geeignete Behälter abfüllt. Das Krestin kann man dabei in einer eine Konzentration von etwa 0.5 bis etwa 2%, insbesondere etwa 1% ergebenden Menge verwenden.

Das stickstoffhaltige Polysaccharid Krestin, das auch als PSK bezeichnet wird, wurde von Forschern der Firma Kureha Chemical Industry Co., Ltd. Tokio, aus Myzelien von Pilzen des Genus Coriolus, insbesondere von Coriolus versicolor (Fr.) Qel (Basidiomyceten, Familie Polyporaceae) mit Wasser in der Wärme extrahiert, nachdem Anlass zur Annahme bestanden hatte, diese Myzelien enthielten tumorhemmende Stoffe.

Die Gewinnung eines stickstoffhaltigen Polysaccharid-Gemisches durch Extraktion wurde in der Japanischen Auslegeschrift 75/36322 (angemeldet 3.10.68), und die Fraktionierung der Extrakte mittels Bariumhydroxid zur Isolierung der gegen Sarcoma 180 wirksamen Anteile in der Japanischen Auslegeschrift 73/8489 (angemeldet 29.9.70) beschrieben. Weitere Patentanmeldungen mit japanischer Priorität vom 18.12.75, wie die Deutsche Offenlegungsschrift 2 655 844, betreffen ein modifiziertes Extraktionsverfahren. Ausführliche Angaben über die Fraktionierung der rohen Extrakte und Isolierung der tumorhemmenden Anteile sowie über Art und Menge der bei der Hydrolyse der verschiedenen Fraktionen erhaltenen Zucker und Aminosäuren machen Susumi Hirase et al. in Yakugaku Zasshi (J. Pharm. Soc. Japan) *96*, 413—418 (1976). Gemäss der anschliessenden Publikation (ibid. *96*, 419—424 (1976)) untersuchten dieselben Autoren die Konstitution der β-D-Glucan-Teile der fraktionierten Polysaccharide. Ueber die wachstumshemmende Wirkung von PSK auf das Sarkom 180 an der Maus und das Ascites-Hepatom AH—13 bei oraler Applikation berichteten Shigeru Tsukagoshi et al. in der Zeitschrift Gann 1974, *65*(6) 557—8 [CA. *82*, 119037a (1975)] und in Prog. Chemother., Proc. 8th Int. Congr. Chemother., 1973 [CA. *84*, 54002e (1976)]. Chemische und insbesondere pharmakologische und klinische Publikationen und Berichte über das hier als PSK bezeichnete tumorhemmende, stickstoffhaltige Polysaccharid sind in der Firmenschrift, "An Outline of PSK", 1977, Kureha Chemical Industries Co. Ltd., Tokio, zusammengestellt und referiert. Gemäss dieser Schrift ist PSK ein braunes oder bräunliches, geschmackloses Pulver mit schwachem, spezifischem Geruch, schwerlöslich in Methanol, Pyridin, Chloroform, Benzol und Hexan, aber löslich in Wasser. Eine 1%ige wässrige Lösung ist bräunlich, mit leichter Trübung, ihr pH-Wert liegt bei 6,6 bis 7,2. Beim Erhitzen zersetzt sich PSK (= Krestin) oberhalb 120°C.

Die prophylaktische Wirkung gegen Viren des Respirationstraktes kann bei den verschiedenen Chargen des gegenwärtig von der Firma Kureha zur Verwendung als Tumorhemmstoff produzierten und in den Handel gebrachten Krestin innerhalb gewisser Grenzen schwanken. Deshalb ist es ratsam, Proben der zu verwendenden Chargen in Mäuse-Infektionsschutzversuchen im voraus zu prüfen und gegebenenfalls auf die Verwendung einzelner Chargen mit schwächerer, z.B. deutlich unterhalb derjenigen der für die nachstehend beschriebenen Versuche verwendeten Chargen liegender Wirksamkeit zu verzichten.

Die prophylaktische Wirkung des Krestins gegen Infektionen durch Influenza A- und Influenza B-Viren geht beispielsweise aus den nachstehend beschriebenen Versuchen hervor. Für sämtliche Versuche wurde Krestin aus den als bezüglich der Influenza-Prophylaxe gut wirksam erkannten Chargen der Firma Kureha, die von ihr als Lot 1228 und Lot 1214 in den Handel gebracht wurden, verwendet.

### Methode

Männliche NMRI-Mäuse von 16 bis 18 g Körpergewicht, eingeteilt in Gruppen von je 10, werden mit einer für 90% der Tiere tödlichen Suspension von Influenza A/Hongkong 1/68-Viren ($H_3N_2$) bzw. Influenza B/Ann Arbor-Viren in 0,05 ml Wasser unter leichter Aethernarkose intranasal infiziert. Zuvor werden die Mäuse der verschiedenen Gruppen, ausgenommen der zwei Kontrollgruppen, durch intranasale Applikation von je 0,05 ml Lösung von Krestin in verschiedenen Konzentrationen in destilliertem Wasser zu verschiedenen, in der Tabelle I angegebenen Zeiten ein- oder mehrmals vorbehandelt. Die Wirkung der Vorbehandlungen wird anhand der Prozentzahl der nach 15 Tagen überlebenden Mäuse im Vergleich zu den Kontrollgruppen, und anhand der Verlängerung der mittleren Ueberlebenszeit innerhalb einer Beobachtungsperiode von 15 Tagen im Vergleich zu den Kontrollgruppen festgestellt. Die Resultate sind in der nachstehenden Tabelle I zusammengestellt.

| K. d. L. 1) | Applikationszeit Tage (D) bzw. Minuten (M) vor Infektion | Schutzwirkung gegen Influenza-Viren | | | |
|---|---|---|---|---|---|
| | | A Hong Kong 1/68 | | B/Ann Arbor | |
| | | Ueberlebende in %, Test/Kontr. | Mittlere Ueberlebenzeit, Tage Test/Kontr. | Ueberlebende in % Test/Kontr. | Mittlere Ueberlebenzeit, Tage Test/Kontr. |
| 40 | D5, D3, M30 | 80/10** | 13,3/8,8** | 80/10** | 13,6/8,7*** |
| 10 | D5, D3, M30 | 80/10** | 13,3/8,8** | 70/10* | 12,9/8,7** |
| 10 | D3, M30 | 89/10** | 14,4/8,8*** | 70/10* | 13,5/10,5*** |
| 10 | M30 | 50/10 | 11,5/9,8 | 20/10 | 10,4/10,5 |
| 10 | D1 | 40/10 | 11,9/9,8 | 20/10 | 11,1/10,5 |
| 10 | D2 | 70/10 | 12,6/9,7 | | |
| 10 | D3 | 80/10** | 13,7/9,8*** | 50/10 | 12,5/10,5** |
| 10 | D4 | 80/10** | 13,7/9,7*** | | |
| 10 | D7 | 80/10** | 14,0/9,7*** | | |
| 10 | D9 | 70/10* | 13,2/9,7*** | | |
| 10 | D11, D9, D7 D4, D2 | 90/10** | 14,2/9,7** | | |
| 4 | D3, M30 | 30/10 | 10,9/9,8 | 60/10 | 13,1/10,5** |
| 1 | D3, M30 | 20/10 | 9,4/9,8 | 50/10 | 12,7/10,5 |

\*    Signifikant P<0,05     Contingency-Test für Ueberlebende bzw.
\*\*    Signifikant P<0,05     Cox-Test für mittlere Ueberlebenzeit
\*\*\* Signifikant P<0,001

1) Konzentration der Lösung in mg/ml

Aus den in der Tabelle I zusammengestellten Resultaten geht hervor, dass Krestin in der Konzentraton von 10 mg/ml = 1% schon bei einmaliger Applikation, wenn diese mindestens 2—3 Tage bis 7 Tage vor der Infektion liegt, eine starke Schutzwirkung gegen Influenza A- und Influenza B- Viren gewährleistet. Niedrigere Konzentrationen, wie z.B. 0,4% sind etwas schwächer wirksam.

Bei der makroskopischen und mikroskopischen Untersuchung der Lungen von Mäusen, denen 7 Tage vor Infektion mit A/HK 1/68 eine 1%ige Krestin-Lösung nasal appliziert wurde, fanden sich deutlich geringere pneumonische Befunde als bei den mit Placebo behandelten Kontrolltieren.

Gleichartige Schutzeffekte wie die vorstehenden gegen Influenzaviren A/Hongkong 1/68 ($H_3N_2$) und B/AA 4/56 fanden sich auch gegen Infektionen mit anderen A ($H_3N_2$)-Stämmen, z.b. A/Vict. 3/75 und A/Port Chalmers 1/73 und insbesondere A/Texas 1/77, mit A ($H_2N_2$)-Stämmen, z.B. A/Singapore 1/57, mit A ($H_1N_1$)-Stämmen, z.B. A/USSR 92/77, wie auch gegen Infektionen mit Influenza B-Viren, wie Influenza B/Lee 40, und Parainfluenza-Viren, wie Parainfluensza-I/Sendai.

Diese Wirkungen des Krestins bei nasaler Verabreichung übertreffen die prophylaktische Wirkung von bekannten antiviral wirksamen Stoffen, wie Amantadin, gegenüber Influenza A-Viren, überdies bietet Krestin überraschenderweise auch einen in der Praxis sehr erwünschten Schutz nicht nur gegen Influenza A-Viron, sondern auch gegen Infektionen durch Influenza B-Viren und andere respiratorische Viren. Bei oraler oder subcutaner Verabreichung von Krestin kann dagegen keine signifikante Schutzwirkung erreicht werden, wie Versuche an in oben angegebener Weise

durch Influenza A/Hongkong 1/68 ($H_3N_2$) infizierten Mäusen ergeben haben.

Wie entsprechende Versuche gezeigt haben, hemmt die Prophylaxe mit Krestin die Bildung hämagglutinierender Serum-Antikörper gegen das Virus nicht. In Versuchen mit Influenzavirus-Infektionen von Gewebekulturen zeigt Krestin keine Hemmung der Plaque-Bildung und ist somit nicht antiviral im Sinne von z.B. Amantadin wirksam. Dagegen war nach Mischung einer Suspension von A/HK 1/68 (10 × $LD_{90}$ pro 0,05 ml) mit einer 1-% Krestin-Lösung in vitro und 30 Minuten Verweilzeit der Mischung bei 23°C die Infektiosität bei nasaler Applikation an der Maus aufgehoben, der Hämagglutinations-Hemmtiter im Serum war stark erhöht und die mit dieser Mischung behandelten Tiere waren resistent gegen eine Reinfektion mit der $LD_{90}$ desselben Virus.

Bei gleichzeitiger subcutaner Applikation einer Vakzine gegen A/Vict. 3/75 (Begrivac®, geschützte Marke der Behringwerke, Marburg a.d. Lahn, BR Deutschland) und nasaler Applikation einer 1% igen Lösung von Krestin erwiesen sich die Mäuse in der Antikörper-freien Latenzphase gegen Infektion mit dem gleichen Virus als geschützt.

Die lokale Verträglichkeit wässriger Lösungen von Krestin in niedriger Konzentration ist gut. Am Kaninchenauge führte die zweimalige tägliche Anwendung der 1%-igen wässrigen Lösung während 4 Tagen zu keiner Reizung, was umso beachtlicher ist, als in der praktischen Anwendung mehrtägige Behandlungsintervalle möglich sind.

Die nasale Applikation von 0,1 ml 1%- oder 3%-iger Lösungen an Meerschweinchen, drei- oder fünfmal pro Woche während dreimal 4 Wochen, führte zu keinen allergischen Symptomen oder Bildung von Serum-Antikörpern, während die mit dem bekannten Allergen Ovalbumin ebenso behandelten Tiere bereits während oder nach der ersten 4-wöchigen Behandlung an Anaphylaxie starben und Serum-Antikörper aufwiesen.

Krestin besitzt keine cytotoxische oder cytostatische Wirksamkeit und wurde bei seiner klinischen Prüfung als tumorhemmender Stoff auch bei oraler Verabreichung in höheren Dosen von üblicherweise 1 g und mehr pro Tag gut vertragen.

Aufgrund der tumorhemmenden Wirksamkeit von Krestin bei oraler Verabreichung konnte keineswegs vorausgesehen werden, dass Krestin bei nasaler Verabreichung die erfindungsgemäss festgestellte Schutzwirkung gegen Virusinfektionen ausüben würde. Durch die bekannte orale Verabreichung von Krestin in relativ hohen Dosen als Tumorhemmstoff wurde auch dessen nasale Verabreichung zum gleichen Zweck und die Herstellung entsprechender Präparate in keiner Weise nahegelegt.

Die erfindungsgemässen Nasalpräparate sind insbesondere mit üblichen Hilfsstoffen bereitete Nasaltropfen, Nasalsprays, Nasalgele und Nasalsalben. Diese enthalten den Wirkstoff in einer prophylaktisch wirksamen, d.h. eine Schutzwirkung gegen Virusinfektionen der oben besagten Art gewährleistenden Menge und Konzentration. Entsprechend enthalten sie den Wirkstoff in einer für eine Verabreichung von mindestens 2 mg Wirkstoff pro Mal geeigneten Konzentration. Die obere Grenze der letzteren liegt bei 4% (G/V) und die untere Grenze, — weil eine einzelne Verabreichung nicht nur durch einmalige nasale Applikation, sondern auch durch nach Antrocknen ein- oder mehrmals wiederholte Applikation erfolgen kann — bei etwa 0,2% (G/V), entsprechend 1 ml eines gebrauchsfertigen Nasalpräparates für die obige Mindestdosis. Nasaltropfen enthalten insbesondere zwischen 1 und 2%, und Nasalsprays insbesondere 0,2 bis 1% Wirkstoff. In beiden Fällen dient als Lösungsmittel vorzugsweise Wasser; die wässrigen Lösungen enthalten gegebenenfalls übliche pharmazeutisch annehmbare Zusätze zur Stabilisierung des Wirkstoffes, Pufferung, Konservierung und/oder Herabsetzung der Oberflächenspannung und können in üblicher Weise, z.B. mit Natriumchlorid oder Pufferlösungen, isotonisch gemacht sein.

Die Erfindung betrifft in erster Linie mit obengenannten Lösungen gefüllte Sprayfläschchen und ähnliche zur intranasalen Applikation solcher Lösungen geeignete Behälter.

Die Anwendung der erfindungsgemässen Mittel zur Prophylaxe gegen Influenza A, Influenza B und Infektionen durch andere Viren des Respirationsstraktes erfolgt vorzugsweise in Abständen von 2 bis 3 Tagen bzw. zweimal wöchentlich. Statt für eine länger, z.B. die ganze kalte Jahreszeit dauernde Prophylaxe können die erfindungsgemässen Mittel auch nur während eines Teiles der kalten Jahreszeit bei offensichtlich erhöhter Ansteckungsgefahr angewendet werden. Weiter können sie auch zur Ueberbrückung der Latenzphase bei Influenza-Schutzimpfungen, d.h. gleichzeitig mit oder unmittelbar vor der Impfung, angewendet werden, da sie bei gleichzeitiger nasaler Applikation mit parenteral injizierter Vakzine einen Schutz in der Antikörper-freien Latenzphase gewähren, ohne die Antikörperbildung zu beeinträchtigen. Dadurch kann das Risiko einer Infektion innerhalb der ersten beiden Wochen nach der Vakzinierung vermindert werden.

Aus der weiter oben erwähnten Eigenschaft des Krestins, die Infektiosität von Virussuspensionen, aber nicht deren Fähigkeit zur Induktion der Antikörperbildung aufzuheben ergibt sich weiter die Möglichkeit, Nasalpräparate zur Prophylaxe gegen Infektionen durch Viren des Respirationstraktes mit Vakzinewirkung herzustellen. Solche Präparate enthalten in wässrigem medium pro 0,5 ml, d.h. in der durch einmalige Applikation von je 0,25 ml in jede Nasenhöhle bequem verabreichbaren Menge Flüssigkeit 2,5 bis 10 mg, insbesondere etwa 5 mg, entsprechend einer Konzentration von 0,5 bis 2%, vorzugsweise 1,0%, Krestin gemischt mit mindestens einem Stamm von Viren des Respirationstraktes in einer Menge, die diejenige nicht übertrifft, welche durch Einwirkung des anwesenden Krestins bei Raum- bis Körpertemperatur, insbesondere bei 20 bis 25°C, nicht-infektiös wird, und gegebenenfalls übliche Zusatzstoffe, wie z.B. Konservierungsmittel. Zur Herstellung solcher Präparate mischt man eine wässrige Lösung des Krestins von geeigneter Konzentration mit einer wässrigen Suspension mindestens eines Stammes von Viren des Respirationstraktes. Dabei werden die Konzentrationen der wässrigen Lösung von Krestin und der Virus-Suspension derart gewählt, dass die erhaltene Mischung pro 0,5 ml 2,5 bis 10 mg Krestin, und insbesondere etwa 5 mg Krestin, und die Viren in einer Menge enthält, die diejenige nicht übertrifft, welche durch die Einwirkung des anwesenden Krestins bei Raum- bis Körpertemperatur nicht-infektiös werden kann. Die Zeitdauer der Einwirkung bei gegebener oder ebenfalls variierender Einwirkungstemperatur wird in Mäuse-Infektionsschutzversuchen mit einer entsprechenden Probemischung ermittelt und liegt im bevorzugten Temperaturbereich von 20—25°C, insbesondere bei 23°C, zwischen etwa 15 Minuten und etwa 120 Minuten je nach der Empfindlichkeit des betreffenden Virus. Für A/Hongkong 1/68 ($H_3N_2$) ist die Einwirkungsdauer z.B. kürzer als für A/Texas 1/77. Anschliessend fügt man gewünschtenfalls übliche Zusatzstoffe, z.B. Konservierungsmittel, wie Natriumtimerfonat [Natrium-p-(äthylmercurithio)-benzolsulfonat], zu und bewahrt das Präparat bis zum Gebrauch im Kühlschrank auf. Die Anwendung dieser erfindungsgemässen Mittel erfolgt ähnlich wie diejenige der Mittel ohne Vakzinewirkung, jedoch als bloss einmalige Applikation für eine längere Zeitspanne, z.B. jeweils bei Beginn der kalten Jahreszeit.

Nachstehend folgen Beispiele für Applikationsformen. Diese Beispiele sollen jedoch den Umfang der Erfindung in keiner Weise beschränken.

### Beispiel 1
*Nasalspray*

Zur Bereitung eines Nasalsprays mit 1% Wirkstoffgehalt löst man 10,0 g Krestin in 1 Liter destilliertem Wasser und füllt die Lösung, gewünschtenfalls nach Zusatz von 10 mg Natrium-timerfonat [Natrium-p-(äthylmercurithio)-benzolsulfonat], in Spray-Fläschchen von z.B. 10 ml Inhalt. Zur Prophylaxe gegen Influenza und andere Infektionen durch Viren des Respirationstraktes werden z.B. jeden 2. oder 3. Tag oder zweimal wöchentlich aus vollem Fläschchen 2—3 Druckstösse, aus teilweise entleertem Fläschchen nötigenfalls mehr, in jede Nasenhälfte gespritzt.

### Beispiel 2
*Nasaltropfen*

Zur Bereitung von Nasaltropfen mit 1% Wirkstoffgehalt löst man 10,0 g Krestin in 1 Liter destilliertem Wasser und füllt die Lösung, gewünschtenfalls nach Zusatz von 10 mg natrium-timerfonat, in mit einem als Pipette ausgestalteten Stopfen versehene Tropffläschchen von z.B. 5 oder 10 ml Inhalt. In gleichen Intervallen, wie im Beispiel 1 angegeben, appliziert man je 4—6 Tropfen, entsprechend ca. 0,2 oder 0,3 ml enthaltend ca. 2—3 mg Wirkstoff, in jede Nasenhälfte.

### Beispiel 3
*Nasalpräparat mit Vakzinewirkung*

Zur Herstellung von 100 ml eines Nasalpräparates mit Vakzinewirkung, das eine 1%-ige wässrige Lösung von Krestin mit einem Gehalt von ursprünglich 700 infektiösen Einheiten (I E) Influenza-Virus A/Texas 1/79 pro 0,5 ml (übliche Impfdosis) enthält, werden 80 ml einer 1,25%-igen Lösung von Krestin in destilliertem Wasser mit 20 ml einer Suspension von in üblicher Weise gereinigtem Virus A/Texas 1/77 mit einem Gehalt von 7000 I E pro ml vermischt. Die Mischung wird während einer Zeitspanne, die zuvor in Mäuse-Infektionsschutzversuchen ermittelt wurde und in der eine entsprechende Probemischung sich als nicht-infektiös erwies, aber hohe Antikörpertiter und Resistenz gegenüber Reinfektion erzeugte und im vorliegenden Falle 60 Minuten betrug, bei 23°C gehalten. Danach wird gewünschtenfalls ein Konservierungsmittel, z.B. 1,0 g Natriumtimerfonat, zugegeben und das Präparat bis zum Gebrauch im Eisschrank aufbewahrt.

Für die Influenza-Prophylaxe durch Vakzinierung werden, vorzugsweise zu Beginn der kalten Jahreszeit, je 0,25 ml des Präparates mit einer Pipette in jede Nasenhöhle getropft.

### Patentansprüche

1. Nasalpräparate zur Prophylaxe gegen Infektionen durch Viren des Respirationstraktes, dadurch gekennzeichnet, dass sie als Wirkstoff 0,2—4% Krestin enthalten.

2. Nasalpräparate gemäss Anspruch 1 in Form von üblichen Arzneiformen für die intranasale Anwendung.

3. Nasalpräparate gemäss Anspruch 1 in Form von Nasalsprays.

4. Nasalpräparate gemäss Anspruch 1 in Form von Nasaltropfen.

5. Nasalpräparate gemäss Anspruch 1 in Form von Nasalgelen.

6. Nasalpräparate gemäss Anspruch 1 in Form von Nasalsalben.

7. Nasalpräparate gemäss Anspruch 1, bestehend aus einer wässrigen Lösung von 0.2—4% Krestin und gegebenenfalls von überlichen Zusatzstoffen in einem zur nasalen Applikation einer Flüssigkeit geeigneten behälter.

8. Nasalpräparate gemäss Ansprüchen 3 oder 7, gekennzeichnet durch einen Gehalt an etwa 0,2 bis etwa 1% Krestin.

9. Nasalpräparate gemäss Anspruch 4, gekennzeichnet durch einen Gehalt an etwa 1 bis etwa 2% Krestin.

10. Nasalpräparate gemäss einem der Ansprüche 1—4 und 7—9 zur Prophylaxe von Infektionen durch Viren des Respirationstraktes mit Vakzinewirkung, dadurch gekennzeichnet, dass sie in wässrigem Medium pro 0,5 ml 2,5 bis 10 mg Krestin gemischt mit mindestens einem Stamm von Viren des Respirationstraktes in einer Menge, die diejenige nicht übertrifft, welche durch Einwirkung des anwesenden Krestins bei Raum- bis Körpertemperatur nicht-infektiös wird, und gegebenenfalls übliche Zusatzstoffe enthalten.

11. Nasalpräparate gemäss Anspruch 10, dadurch gekennzeichnet, dass sie pro 0,5 ml etwa 5 mg Krestin enthalten.

12. Verfahren zur Herstellung von Nasalpräparaten gemäss einem der Ansprüche 1—9, dadurch gekennzeichnet, dass man Krestin in einer eine Konzentration von 0.2—4% ergebenden Menge in Wasser löst, gegebenenfalls übliche Zusatzstoffe beifügt und gewünschtenfalls die erhaltene Lösung in zur nasalen Applikation einer Flüssigkeit geeignete Behälter abfüllt.

13. Verfahren nach Anspruch 12, dadurch gekennzeichnet, dass man Krestin in einer eine Konzentration von etwa 0,5 bis etwa 2% ergebenden Menge verwendet.

14. Verfahren nach Anspruch 12, dadurch gekennzeichnet, dass man Krestin in einer eine Konzentration von etwa 1% ergebenden Menge verwendet.

15. Verfahren zur Herstellung von Nasalpräparaten gemäss Anspruch 10 oder 11, dadurch gekennzeichnet, dass man eine wässrige Lösung von Krestin mit einer wässrigen Suspension mindestens eines in übilcher Weise gereinigten Stammes von Viren des Respirationstraktes mischt, die Mischung einer Temperatur von Raum- bis Körpertemperatur während einer in Mäuse-Infektionsschutzversuchen mit einer entsprechenden Probemischung ermitteln Zeitspanne, in welcher die Infektiosität der Viren, aber nicht ihre Fähigkeit zur Induktion der Antikörperbildung aufgehoben wird, aussetzt, wobei die Konzentrationen der wässrigen Lösung von Krestin und der Virus-Suspension derart gewählt werden, dass die erhaltene Mischung pro 0,5 ml 2,5 bis 10 mg Krestin und die Viren in einer solchen Menge enthält, die diejenige nicht übertrifft, welche durch die Einwirkung des anwesenden Krestins bei Raum- bis Körpertemperatur nicht-infektiös werden kann, und gegebenenfalls anschliessend übliche Zusatzstoffe zufügt.

16. Verfahren nach Anspruch 15, dadurch gekennzeichnet, dass man die Einwirkung von Krestin auf die Viren bei einer zwischen 20 und 25°C liegenden Temperatur und innerhalb einer in Mäuse-Infketionsschutzversuchen mit einer entsprechenden Probemischung ermitteln, zwischen 15 und 120 Minuten liegenden Zeitspanne durchführt.

17. Verfahren nach Anspruch 15, dadurch gekennzeichnet, dass man pro 0,5 ml der Mischung etwa 5 mg Krestin verwendet.

18. Verwendung von Krestin zur Herstellung von Nasalpräparaten mit einem Krestingehalt von 0.2—4% zur Prophylaxe gegen Infektionen durch Viren des Respirationstraktes.

19. Verwendung von Krestin zur Herstellung von Nasalpräparaten mit einem Krestingehalt von 0.2—4% zur Ueberbrückung der Latenzphase bei Influenza-Schutzimpfungen.

20. Verwendung von Krestin zur Herstellung von Nasalpräparaten mit einem Krestingehalt von 0.2—4% zur Prophylaxe gegen Infektionen durch Viren des Respirationstraktes durch einmalige Applikation für eine längere Zeitspanne.

**Revendications**

1. Préparations nasales destinées à la prophylaxie contre des infections provoquées par des virus des voies respiratoires, caractérisées par le fait qu'elles contiennent 0,2% à 4% de crestine en tant qu'agent actif.

2. Préparations nasales selon la revendication 1, sous forme de médications usuelles pour l'application intranasale.

3. Préparations nasales selon la revendication 1, sous forme de vaporisations nasales.

4. Préparations nasales selon la revendication 1, sous forme de gouttes nasales.

5. Préparations nasales selon la revendication 1, sous forme de gelées nasales.

6. Préparations nasales selon la revendication 1, sous forme de pommades nasales.

7. Préparations nasales selon la revendication 1, se composant d'un mélange d'eau et de 0,2%—4% de crestine et, le cas échéant, des additifs usuels, conditionnées dans un contenant prévu pour l'application nasale d'un liquide.

8. Préparations nasales selon la revendication 3 ou 7, caractérisées par une teneur en crestine d'environ 0,2% à environ 1%.

9. Préparations nasales selon la revendication 4, caractérisées par une teneur en crestine d'environ 1% à 2%.

10. Préparations nasales selon les revendications 1—4 et 7—9 destinées à la prophylaxie d'infections provoquées par des virus des voies respiratoires avec effet vaccinatoire, caractérisées par le fait qu'elles contiennent 2,5 mg à 10 mg de crestine par mélange aqueux de 0,5 ml auquel sont ajoutés, le cas échéant, des additifs usuels et au moins une sorte de virus des voies respiratoires en quantité ne dépassant pas celle qui ne devient pas infectieuse à une température allant de ambiante à la température corporelle grâce à l'effet de la crestine.

11. Préparations nasales selon la revendication 10, caractérisées par une teneur en crestine d'environ 5 mg pour 0,5 ml.

12. Procédé destiné à la fabrication de préparations nasales selon l'une des revendications 1—9, caractérisée par le fait que l'on dissout dans de l'eau de la crestine pour atteindre une concentration de 0,2%—4%, que l'on y ajoute, le cas échéant, des additifs usuels, et, lorsqu'on le souhaite, que l'on conditionne le mélange ainsi obtenu dans un contenant prévu pour l'application nasale d'un liquide.

13. Procédé selon la revendication 12, caractérisée par le fait que l'on utilise la crestine sous une concentration d'environ 0,5% à environ 2%.

14. Procédé selon la revendication 12, caractérisée par le fait que l'on utilise la crestine sous une concentration d'environ 1%.

15. Procédé destiné à la fabrication de préparations nasales selon la revendication 10 ou 11, caractérisé par le fait que l'on mélange une solution aqueuse de crestine à une suspension aqueuse d'au moins une sorte de virus des voies respiratoires purifié de manière usuelle, que l'on met en contact ce mélange à une température allant de l'ambiante à la température corporelle pendant une durée déterminée découlant d'essais de protection contre l'infection réalisés avec un mélange de référence correspondant, cette durée étant telle que l'infectiosité des virus est annhilée, sans que soit affectée leur capacité d'induction de la formation d'anticorps, les concentrations de la solution de crestine et de la suspension de virus étant choisies de sorte que le mélange obtenu contienne 2,5 mg à 10 mg de crestine par 0,5 ml et que les virus y soient en quantité telle qu'elle ne dépasse pas celle qui ne devient pas infectieuse à une température allant de l'ambiante à la température corporelle grâce à l'effet de la crestine présente, mélange auquel sont ajoutés ensuite, le cas échéant, des additifs usuels.

16. Procédé selon la revendication 15, caractérisée par le fait que l'on fait agir la crestine sur les virus à une température de 20° à 25°C pendant une durée découlant d'essais de protection contre l'infection effectués sur des souris, comprise entre 15 et 120 minutes.

17. Procédé selon la revendication 15, caractérisée par le fait que l'on utilise environ 5 mg de crestine par 0,5 ml de mélange.

18. Utilisation de crestine pour la fabrication de préparations nasales d'une teneur en crestine de 0,2%—4% destinées à la prophylaxie contre les infections virales des voies respiratoires.

19. Utilisation de crestine pour la fabrication de préparations nasales d'une teneur en crestine de 0,2%—4% destinées à surmonter la phase latente dans le cas de vaccinations protectrices contre la grippe (influenza).

20. Utilisation de crestine pour la fabrication de préparations nasales d'une teneur en crestine de 0,2%—4% destinées à la prophylaxie des infections virales des voies respiratoires par une unique application sur une période de temps plus longue.

**Claims**

1. Nasal products for the prophylaxis of infections by viruses of the respiratory tract, characterised in that they contain as active compound 0.2—4% of krestin.

2. Nasal products according to Claim 1 in the form of customary drug forms for intranasal administration.

3. Nasal products according to Claim 1 in the form of nasal sprays.

4. Nasal products according to Claim 1 in the form of nasal drops.

5. Nasal products according to Claim 1 in the form of nasal gels.

6. Nasal products according to Claim 1 in the form of nasal ointments.

7. Nasal products according to Claim 1, consisting of an aqueous solution of 0.2—4% of krestin, and, where appropriate, customary additives in a container suitable for nasal administration of a liquid.

8. Nasal products according to Claims 3 or 7, characterised by a content of about 0.2 to about 1% of krestin.

9. Nasal products according to Claim 4, characterised by a content of about 1 to about 2% of krestin.

10. Nasal products according to one of Claims 1—4 and 7—9 for the prophylaxis of infections by viruses of the respiratory tract with a vaccine action, characterised in that they contain in an aqueous medium 2.5 to 10 mg of krestin per 0.5 ml, mixed with at least one strain of viruses of the respiratory tract in an amount which does not exceed that which becomes non-infectious on exposure, at room to body temperature, to the krestin which is present, and, where appropriate, customary additives.

11. Nasal products according to Claim 10, characterised in that they contain as nitrogen-containing polysaccharide about 5 mg of krestin per 0.5 ml.

12. Process for the preparation of nasal products according to one of Claims 1—9, characterised in that krestin is dissolved, in an amount resulting in a concentration of 0.2—4%, in water, where appropriate customary additives are added and, if desired, the resulting solution is dispensed into containers suitable for nasal administration of a liquid.

13. Process according to Claim 12, characterised in that krestin is used in an amount resulting in a concentration of about 0.5 to about 2%.

14. Process according to Claim 12, characterised in that krestin is used in an amount resulting in a concentration of about 1%.

15. Process for the preparation of nasal products according to Claim 10 or 11, characterised in that an aqueous solution of krestin is mixed with an aqueous suspension of at least one strain of viruses of the respiratory tract, which strain has been purified in a customary manner, the mixture is exposed to a temperature from room to body temperature for a time span which has been determined in mouse infection protection tests with a corresponding sample mixture and in which the infectiosity of the viruses, not their ability to induce the formation of antibodies, has been abolished, the concentrations of the aqueous solution of krestin and of the virus suspension being selected such that each 0.5 ml of the resulting mixture contains 2.5 to 10 mg of krestin and that amount of viruses which does not exceed that which can become non-infectious on exposure, at room to body temperature, to the krestin which is present, and, where appropriate, then customary additives are added.

16. Process according to Claim 15, characterised in that the exposure of the viruses to krestin is carried out at a temperature between 20 and 25°C and within a time span which has been determined in mouse infection protection tests with a corresponding sample mixture and is between 15 and 120 minutes.

17. A process according to Claim 15, characterised in that about 5 mg of krestin is used per 0.5 ml of the mixture.

18. Use of krestin for the manufacture of nasal products containing 0.2—4% thereof for the prophylaxis of infections by viruses of the respiratory tract.

19. Use of krestin for the manufacture of nasal products containing 0.2—4% thereof for bridging over the latency phase with protective inoculations against influenza.

20. Use of krestin for the manufacture of nasal products containing 0.2—4% thereof for the prophylaxis of infections by viruses of the respiratory tract by single administration for a prolonged time span.